# EUROPEAN PATENT SPECIFICATION

(11) **EP 1 191 009 B1**
(45) Date of publication and mention of the grant of the patent: **24.11.2004**
(21) Application number: 00942366.6
(22) Date of filing: 28.06.2000
(51) Int. Cl.: C07C 29/44, C07C 31/38, B41M 5/26, G11B 7/26, C07C 29/62

(54) **PROCESS FOR PRODUCING FLUOROALKANOL**
VERFAHREN ZUR HERSTELLUNG VON FLUORALKANOL
PROCEDE DE PRODUCTION DE FLUOROALCANOL

(30) Priority: 30.06.1999 JP 18570199
(43) Date of publication of application: 27.03.2002
(73) Proprietor: ASAHI GLASS COMPANY LTD., Tokyo 100-8405 (JP)
(72) Inventor: WADA, Akihiro, Ichihara-shi, Chiba 290-0058 (JP); TANAKA, Hidemi, Ichihara-shi, Chiba 290-0058 (JP); TANABE, Koichiro, Ihihara-shi, Chiba 290-0058 (JP); YAMAGISHI, Nobuyuki, Ichihara-shi, Chiba 290-0058 (JP); TOMA, Toshihiko, Ichihara-shi, Chiba 290-0058 (JP)
(74) Representative: Müller-Boré & Partner Patentanwälte
(86) International application number: PCT/JP2000/004248
(87) International publication number: WO 2001/002329

(56) References cited:
- JP-A- 4 008 585
- US-A- 3 022 356
- US-A- 5 227 540
- PATENT ABSTRACTS OF JAPAN & JP 05 258346 A (CANON INC.), 8 October 1993 (1993-10-08)

## Description

### TECHNICAL FIELD

The present invention relates to a process for producing a fluoroalkanol. The fluoroalkanol obtainable by the process of the present invention is a compound useful as a starting material for a solvent for the production of an information recording medium, a water and oil repellent, a surface active agent and a color developing material for photographs.

### BACKGROUND ART

A fluoroalkanol is useful as a starting material for a water and oil repellent, a surface active agent, a color developing material for photographs, etc. (JP-A-54-154707). Further, it does not dissolve a plastic substrate made of e.g. polycarbonate, so that it is useful as a solvent for e.g. a dye for an optical recording material in the production of an information recording medium having a recording layer capable of writing in and reading out information by a laser, formed on a substrate (JP-A-4-8585, JP-A-5-258346).

Heretofore, as a method for producing a fluoroalkanol of the following formula 4 (wherein m is an integer of at least 1), (1) a method of charging methanol, tetrafluoroethylene and a radical initiator all at once and heating them (U.S. Patent 2,559,628), (2) a method of charging methanol, tetrafluoroethylene and a radical initiator all at once and reacting them continuously in a reaction tower (JP-B-42-20782, U.S. Patent 3,022,356) and (3) a method of charging methanol and a radical initiator all at once and adding tetrafluoroethylene continuously for reaction (JP-A-54-154707), have been known.

H-(CF₂CF₂)ₘ-CH₂-OH Formula 4

However, in the method (1) it is difficult to control the amount of addition of tetrafluoroethylene, whereby even if it is attempted to obtain a useful object having a value m of from 1 to 4 only, the molecular weight distribution of the product tends to be wide, and the yield tends to be low, such being a problem. Further, the method (2) has a problem that the concentration of the resulting product is low, and the efficiency is accordingly poor, a high pressure condition is required, and it is hardly industrially practical. In the method (3), an acid binding agent is added to the reaction system in order to prevent deterioration of the selectivity due to slowing down of the reaction as the time passes. The acid-binding agent is usually a solid powder such as magnesium oxide, calcium oxide or calcium carbonate, and if it is added to the system, there will be a problem such that it takes time for its separation.

US 3 022 356 discloses the production of fluoroalkanols by reaction of an alkanol and a perfluoroolefin in the presence of a radical initiator which is added at the initial state of the process by one time.

Further, a fluoroalkanol obtainable by a conventional method has had a problem that even if it is subjected to distillation, an evaporation residue will remain, and the purity is not necessarily high, whereby when used as a solvent in the production of the above-mentioned information recording medium, it is not necessarily suitable for the production of an information recording medium of high quality, due to an influence of impurities.

### DISCLOSURE OF THE INVENTION

The present invention is the following invention made for the purpose of solving the above problems and providing a process for producing a fluoroalkanol of high purity having no evaporation residue remained, which is advantageous for an industrial operation with the conversion and selectivity improved.

Namely, the present invention provides a process for producing a fluoroalkanol of the following formula 1, characterized in that in a reaction of reacting an alkanol of the following formula 2 with a perfluoroolefin of the following formula 3 to produce a fluoroalkanol of the following formula 1, the reaction is carried out while continuously adding a radical initiator and a perfluoroolefin of the following formula 3:

CHR¹R²-OH Formula 2

CF₂=CFR³ Formula 3

H-(CFR³CF₂)ₙ-CR¹R²-OH Formula 1

provided that the symbols in the formulae have the following meanings:
R¹, R²: each independently a hydrogen atom or a C₁₋₃ alkyl group,
R³: a fluorine atom or a C₁₋₄ perfluoroalkyl group, and
n: an integer of from 1 to 4.

### BEST MODE FOR CARRYING OUT THE INVENTION

In the formula 2, each of R¹ and R² which are independent of each other, is a hydrogen atom or a C₁₋₃ alkyl group, and each is preferably a hydrogen atom or a methyl group. The alkanol of the formula 2 is preferably methanol, ethanol or isopropyl alcohol.

R³ in the formula 3 is a fluorine atom or a C₁₋₄ perfluoroalkyl group, preferably a fluorine atom or a trifluoromethyl group. The perfluoroolefin of the formula 3 is preferably, for example, tetrafluoroethylene or hexafluoropropylene.

In the process of the present invention, the alkanol (formula 2) and the perfluoroolefin (formula 3) are reacted to obtain the fluoroalkanol (formula 1).

In the formula 1, n is an integer of from 1 to 4, preferably 1 or 2, particularly preferably 1. R¹ and R² correspond to R¹ and R² in the alkanol (formula 2) reacted, and R³ is a fluorine atom or a C₁₋₄ perfluoroalkyl group and correspond to R³ in the perfluoroolefin (formula 3) reacted.

In a case where a perfluoroolefin (formula 3) wherein R³ is a C₁₋₄ perfluoroalkyl group, is employed, it is expected that a fluoroalkanol of the following formula 5 may also form. However, usually, due to a steric hindrance of a perfluoroolefin compound, the compound (formula 1) of the present invention will be the only product, or the compound (formula 1) will be the main product.

H-(CF₂CFR³⁰)ₙ-CR¹R²-OH Formula 5

In the formula, n, R¹ and R² have the meanings as described above, and R³⁰ is a C₁₋₄ perfluoroalkyl group. The following compound may be mentioned as specific examples of the fluoroalkanol (formula 1).

H (CF₂)₂CH₂OH,

H (CF₂)₄CH₂OH,

HCF₂CF₂CH (CH₃)OH,

HCF₂CF₂C (CH₃) ₂ OH,

CF₃CHFCF₂CH₂OH,

CF₃CHFCF₂CH(CH₃) OH,

CF₃CHFCF₂C (CH₃)₂OH.

In the reaction of the present invention, the reaction is carried out while continuously adding a radical initiator and a perfluoroolefin (formula 3).

As the radical initiator, it is preferred to employ an organic free radical initiator. As the organic free radical initiator, a peroxide or an azo compound is preferred. Particularly preferred is an alkyl hydroperoxide, a dialkyl peroxide, a peroxy ketal, a diacyl peroxide, a peroxy carboxylate, a peroxy carboxylic acid or a peroxy carbonate.

The following compounds may, for example, be mentioned as the radical initiator. 1,1-bis(tert-butylperoxy)-3,3-5-trimethylcyclohexane, 1,1-bis(tert-butylperoxy)cyclohexane, tert-butylperoxyisopropyl carbonate, tert-butylperoxy isobutylate, tert-butylperoxy pivalate, di-tert-butyl peroxide and tert-butyl hydroperoxide. As the radical initiator, a dialkyl peroxide is preferred which has a particularly high radical-forming ability from the alkanol (formula 2). Particularly preferred is di-tert-butyl peroxide.

In the present invention, the amount of addition of the perfluoroolefin (formula 3) can be easily controlled by continuously adding the radical initiator. Further, in a conventional reaction, there has been a problem that an acid component will be formed during the reaction, whereby the reaction tends to hardly proceed. Whereas, by supplying the radical initiator continuously, formation of the acid content can be suppressed, which is advantageous. Thus, the method of the present invention is an industrially advantageous method wherein the reaction proceeds even in the absence of an acid binding agent.

The total amount of the radical initiator to be added, is preferably from 0.0001 to 0.1 time by mol, particularly preferably from 0.001 to 0.05 time by mol, to the alkanol (formula 2). The total amount of the perfluoroolefin (formula 3) to be added, is preferably from 0.01n to 1.2n time by mol, particularly preferably from 0.05n to 0.5n time by mol, to the alkanol (formula 2).

The operation of continuously adding the radical initiator and the perfluoroolefin (formula 3) to the reaction system, is preferably carried out by the following method. Namely, in a batch system reactor, the alkanol (formula 2) is charged, and then, the radical initiator is supplied by a pump while heating, and at the same time, the perfluoroolefin (formula 3) is supplied. The reaction of the alkanol (formula 2) with the perfluoroolefin (formula 3) is a so-called telomerization reaction. The telomerization reaction is a reaction wherein the radical initiator is decomposed to form a radical, and the radical withdraws a hydrogen atom on carbon of the alkanol (formula 2) to form an alkanol radical, to which the perfluoroolefin (formula 3) will add. And, the method of the present invention is a method capable of controlling the number of addition of the perfluoroolefin (formula 3) in the telomerization reaction which is a chain reaction. Therefore, it is an excellent method, whereby a desired fluoroalkanol (formula 1) can be selectively produced.

The reaction conditions are changed by the value n in the desired product (formula 1). In a case where it is intended to obtain a desired product wherein n is 1, the reaction temperature may be decided by the temperature at which half life of the employed radical initiator is measured. And it is preferably the temperature, at which half life of the employed radical initiator is 10 hours, ±30°C. For example, in a case where di-tert-butyl peroxide (the temperature at which half life is 10 hours: 125°C) is used, it is preferably within a range of from 95 to 155°C, and as an industrially advantageous condition, it is preferably within a range of from 100 to 140°C. The reaction time is preferably from 1 to 40 hours, particularly preferably from 5 to 25 hours. The reaction pressure (gauge pressure) is preferably from 0.2 to 1.2 MPa, particularly preferably from 0.5 to 1.0 MPa.

Further, in a case where it is intended to obtain a desired product wherein n is 2 or more, it is preferred that the reaction temperature is set to be the temperature, at which half life of the radical initiator is 10 hours, ±30°C, and the reaction time is set to be longer than the case where n is 1, particularly preferably from 10 to 100 hours, especially preferably from 25 to 100 hours. The reaction pressure (gauge pressure) is also preferably set to be higher than the case where n is 1, particularly preferably from 0.5 to 5.0 MPa, especially preferably from 0.9 to 2.0 MPa.

Further, with respect to the conditions for supplying the radical initiator, it is preferred to supply from 15 to 30% of the total supply amount within one hour after the initiation of the reaction at a constant speed and continue to supply the remaining amount at a constant speed during the remaining reaction time, irrespective of the value n of the desired product. Further, also with respect to the fluoroolefin, it is preferred to continue to supply the necessary total amount of supply at a constant speed during the reaction time.

By the method of the present invention, the amount of addition of the perfluoroolefin can be controlled, and the fluoroalkanol (formula 1) can be obtained at high selectivity. The obtained fluoroalkanol can be made to have a high purity to such an extent that no evaporation residue will remain, by carrying out a usual separation and purification. The fluoroalkanol (formula 1) is useful as a starting material for e.g. a water and oil repellent, a surface active agent and a color developing material for photographs.

The fluoroalkanol obtained by the present invention, can be made to have a high purity to such an extent that no evaporation residue will remain, and it is particularly useful as a solvent for the production of an information recording medium having a recording layer capable of writing in and/or reading out information by a laser, formed on a substrate.

Such an information recording medium can be produced by using a solvent containing the fluoroalkanol (formula 1) obtained in the present invention, preferably by dissolving a dye in a solvent containing such fluoroalkanol, and coating and drying the obtained solution on a substrate in accordance with a usual method to form a recording layer containing the dye. As such a dye, a cyanine type, phthalocyanine type, pyrylinium type, thiopyrylinium type, squarilium type, azulenium type, indophenol type, indoaniline type, triphenylmethane type, quinone type, aluminum type, diimmonium type or metal complex salt type dye may be mentioned.

As the substrate for such an information recording medium, a plastic such as polycarbonate, polymethyl methacrylate, an epoxy resin, amorphous polyolefin, polyester or polyvinyl chloride, glass or ceramics, may be mentioned. Further, an undercoating layer may be formed between the recording layer and the substrate for the purpose of e.g. improvement of the surface flatness, improvement of adhesiveness and prevention of deterioration of the recording layer, and a protective layer or the like may be formed on the recording layer.

### EXAMPLES

Now, the present invention will be described in detail, but the present invention is by no means restricted by such specific Examples.

### EXAMPLE 1: Preparation of 2,2,3,4,4,4-hexafluorobutanol

Into a 1 ℓ hastelloy C autoclave, 408 g of methanol was charged, and the internal temperature was adjusted to be 125°C. By means of a metering pump, a solution having 8.2 g of di-tert-butyl peroxide dissolved in 72 g of methanol, was supplied for 0.5 hour at a rate of 40 g/hr and thereafter supplied for 15 hours at a rate of 4 g/hr. Further, at the same time, by means of a metering pump, hexafluoropropylene was supplied for 12 hours at a rate of 10.4 g/hr (the total amount of hexafluoropropylene supplied: 125 g). After completion of the supply of the methanol solution of di-tert-butyl peroxide, the mixture was maintained at 125°C for 3 hours and then cooled to 40°C. The reaction solution was analyzed by gas chromatography, whereby the conversion of methanol was 15%, and 2,2,3,4,4,4-hexafluorobutanol was found to have formed at a selectivity of 95%. Further, 2-difluoromethyl-2,3,3,3-tetrafluoropropanol was found to have formed at a selectivity of 5%.

### EXAMPLE 2: Preparation of 2,2,3,3-tetrafluoropropanol

Into a 1 ℓ hastelloy C autoclave, 408 g of methanol was charged, and into the gas phase portion, tetrafluoroethylene was charged, whereupon the internal temperature was adjusted to 125°C. By means of a metering pump, a solution having 8.2 g of di-tert-butyl peroxide dissolved in 72 g of methanol, was supplied for 0.5 hour at a rate of 40 g/hr, and thereafter, supplied for 15 hours at a rate of 4 g/hr. Further, the pressure of tetrafluoroethylene was set to be 0.8 Pa (gauge pressure) by an automatic valve and tetrafluoroethylene was supplied in an amount corresponding to reduction by the reaction. The total amount of tetrafluoroethylene supplied was 230 g. After cooling to 40°C, the reaction solution was analyzed by gas chromatography, whereby the conversion of methanol was 22%, and 2,2,3,3-tetrafluoropropanol was found to have formed at a selectivity of 96%.

### COMPARATIVE EXAMPLE 1: Preparation of 2,2,3,3-tetrafluoropropanol

Into a 1 ℓ hastelloy C autoclave, 800 g of methanol, 14.6 g of di-tert-butyl peroxide and 1.12 g of calcium oxide were charged all at once, and into the gas phase portion, tetrafluoroethylene was charged, and the internal temperature was adjusted to 125°C. Tetrafluoroethylene was supplied over a period of 20 hours in an amount corresponding to reduction by the reaction, under a constant pressure of 0.8 Pa (gauge pressure) by an automatic valve. The total amount of tetrafluoroethylene supplied was 180 g. After cooling to 40°C, the reaction solution was analyzed by gas chromatography, whereby the conversion of methanol was 6.8%, and 2,2,3,3-tetrafluoropropanol was found to have formed at a selectivity of 95%.

### INDUSTRIAL APPLICABILITY

According to the present invention, the fluoroalkanol (formula 1) can be produced at a higher conversion and higher selectivity than ever. The method of the present invention can be carried out without employing any special reaction condition or any special reaction operation, and without employing an acid binding agent. Thus, it is a useful method which is advantageous for an industrial operation.

Further, the fluoroalkanol obtained by the present invention can be made to have a high purity to such an extent that no evaporation residue will remain, and it is particularly suitable as a solvent for the production of an information recording medium having a recording layer capable of writing in and/or reading out information by a laser, formed on a substrate. Thus, the present invention also provides an information recording medium having a recording layer capable of writing in and/or reading out information by a laser, formed on a substrate, which is produced by means of the fluoroalkanol (formula 1).

Further, the fluoroalkanol obtained by the present invention, is useful as a starting material for e.g. a water and oil repellent, a surface active agent and a color developing material for photographs.

## Claims

1. A process for producing a fluoroalkanol of the following formula 1, **characterized in that** in a reaction of reacting an alkanol of the following formula 2 with a perfluoroolefin of the following formula 3 to produce a fluoroalkanol of the following formula 1, the reaction is carried out while continuously adding a radical initiator and a perfluoroolefin of the following formula 3:
CHR¹R²-OH Formula 2
CF₂=CFR³ Formula 3
H-(CFR³CF₂)ₙ-CR¹R²-OH Formula 1
provided that the symbols in the formulae have the following meanings:
R¹, R²: each independently a hydrogen atom or a C₁₋₃ alkyl group,
R³: a fluorine atom or a C₁₋₄ perfluoroalkyl group, and
n: an integer of from 1 to 4.

2. The process according to Claim 1, wherein n is 1.

3. The process according to Claim 1 or 2, wherein the radical initiator is a dialkyl peroxide.

4. The process according to Claim 1, 2 or 3, wherein the alkanol of the formula 2 is methanol or ethanol.

5. The process according to Claim 1, 2, 3 or 4, wherein the perfluoroolefin of the formula 3 is tetrafluoroethylene or hexafluoropropylene.

6. The process according to Claim 1, 2, 3, 4 or 5, wherein the reaction is carried out in the absence of any acid binding agent.

## Patentansprüche

1. Verfahren zur Herstellung eines Fluoralkanols der folgenden Formel 1, **dadurch gekennzeichnet, dass** in einer Reaktion des Umsetzens eines Alkanols der folgenden Formel 2 mit einem Perfluorolefin der folgenden Formel 3 zur Erzeugung eines Fluoralkanols der folgenden Formel 1 die Reaktion durchgeführt wird, während kontinuierlich ein radikalischer Initiator und ein Perfluorolefin der folgenden Formel 3 zugegeben werden:
CHR¹R²-OH Formel 2
CF₂=CFR³ Formel 3
H-(CFR³CF₂)ₙ-CR¹R²-OH Formel 1
mit der Maßgabe, dass die Symbole in den Formeln die folgenden Bedeutungen aufweisen:
R¹, R²: jeweils unabhängig ein Wasserstoffatom oder ein C₁₋₃ Alkylrest,
R³: ein Fluoratom oder ein C₁₋₄ Perfluoralkylrest und
n: eine ganze Zahl 1 bis 4.

2. Verfahren nach Anspruch 1, worin n 1 ist.

3. Verfahren nach Anspruch 1 oder 2, worin der radikalische Initiator ein Dialkylperoxid ist.

4. Verfahren nach Anspruch 1, 2 oder 3, worin das Alkanol der Formel 2 Methanol oder Ethanol ist.

5. Verfahren nach Anspruch 1, 2 , 3 oder 4, worin das Perfluorolefin der Formel 3 Tetrafluorethylen oder Hexafluorpropylen ist.

6. Verfahren nach Anspruch 1, 2, 3, 4 oder 5, worin die Reaktion in der Abwesenheit von jedwedem säurebindenden Mittel durchgeführt wird.

## Revendications

1. Procédé de production d'un fluoroalcanol de formule 1 suivante, **caractérisé en ce que**, dans une réaction consistant à faire réagir un alcanol de formule 2 suivante avec une perfluorooléfine de formule 3 suivante afin de produire un fluoroalcanol de formule 1 suivante, la réaction est effectuée en ajoutant en continu un amorceur radicalaire et une perfluorooléfine de formule 3 suivante :
CHR¹R²-OH Formule 2
CF₂=CFR³ Formule 3
H- (CFR³CF₂)ₙ-CR¹R²-OH Formule 1
avec la réserve que les symboles dans les formules aient les significations suivantes :
R¹, R² : chacun indépendamment un atome d'hydrogène ou un groupe alkyle en C₁-C₃ ;
R³ : un atome de fluor ou un groupe perfluoroalkyle en C₁-C₄ ; et
n : un entier de 1 à 4.

2. Procédé selon la revendication 1, dans lequel n vaut 1.

3. Procédé selon la revendication 1 ou 2, dans lequel l'amorceur radicalaire est un peroxyde de dialkyle.

4. Procédé selon la revendication 1, 2 ou 3, dans lequel l'alcanol de formule 2 est le méthanol ou l'éthanol.

5. Procédé selon la revendication 1, 2, 3 ou 4, dans lequel la perfluorooléfine de formule 3 est le tétrafluoroéthylène ou l'hexafluoropropylène.

6. Procédé selon la revendication 1, 2, 3, 4 ou 5, dans lequel la réaction est effectuée en l'absence de tout agent de fixation d'acide.
